# EUROPEAN PATENT APPLICATION

(11) **EP 3 888 560 A1**
(43) Date of publication of application: **06.10.2021**
(21) Application number: 20167736.6
(22) Date of filing: 02.04.2020
(51) Int. Cl.: A61B 8/08, A61B 5/0402

(54) **MEDICAL SENSING SYSTEM AND POSITIONING METHOD**

(71) Applicant: Koninklijke Philips N.V., 5656 AG Eindhoven (NL)
(72) Inventor: IN 'T GROEN, Robertus Leonardus Maria, 5656 AE Eindhoven (NL); GIJSBERS, Gerardus Henricus Maria, 5656 AE Eindhoven (NL); ZUO, Fei, 5656 AE Eindhoven (NL); JOHNSON, Mark, 5656 AE Eindhoven (NL); CHAKRABARTI, Biswaroop, 5656 AE Eindhoven (NL); HIWALE, Sujitkumar, 5656 AE Eindhoven (NL); BERA, Deep, 5656 AE Eindhoven (NL)
(74) Representative: Philips Intellectual Property & Standards

(57) **Abstract**

A system (10) is for performing ECG measurements and comprises a probe (12) with an integrated one or more ECG electrodes (22) and an ultrasound sensing means (18), such as a transducer arrangement. The probe thus provides a mobile ECG electrode which can be sequentially moved between a set of different locations on the body for acquiring ECG measurements from different angles relative to the heart (i.e. different 'leads'). Positioning of the probe in each required location is guided by a position guidance function which uses ultrasound data acquired by the ultrasound sensing means to locate the probe (with reference to an ultrasound body atlas (28) or map), and uses a stored set of reference body locations to guide a user with guidance information as to how to move the probe to arrive at a next target electrode location. In examples, the user may be guided through a sequence of electrode locations, with ECG data acquired at each one, thereby sequentially building up a set of standard ECG lead measurements.

## Description

### FIELD OF THE INVENTION

This invention relates to a medical sensing system, in particular to system comprising a moveable ECG electrode, and means for guiding positioning of the ECG electrode.

### BACKGROUND OF THE INVENTION

A common type of clinical patient examination is cardiovascular examination.

A cardiovascular examination often comprises examination not only of a patient's heart, but also examination of other parts of the body including the hands, face and neck. The cardiovascular examination aims to identify any cardiovascular pathology that may be causing a patient's symptoms, such as chest pain, breathlessness, or heart failure.

Key observations which may be performed during the physical examination of the heart include: measurement of heart rate; measurement of a size of the heart (e.g. measured by percussion and feeling the ictus of the heart) for instance as an indication for enlargement of the left ventricle; and inspection of heart valve function and blood flow, for instance observed via auscultation of the heart at four standard positions, related to the different heart valves, these being the mitral valve, aortic valve, tricuspid valve, and pulmonary valve. Heart sounds and murmurs give indications for valve defects, volume overload, pressure overload and hypertrophy.

One modality which can be used for performing cardiovascular examination is echocardiography.

An echocardiogram is an ultrasound test which can be used for evaluating structures of the heart, as well as the direction of blood flow within the heart. Technicians specially trained in echocardiography perform the scan using an ultrasound probe to produce images and videos, often using a special probe or transducer that is placed in various places on the chest wall, to view the heart from different directions. Cardiologists, or heart specialists, are trained to evaluate the acquired images to assess heart function and provide a report of the results.

Information produced by an echocardiogram may provide indication of one or more of the following:
- Heart size. Weakened or damaged heart valves, high blood pressure, or other diseases can cause the chambers of the heart to enlarge or the walls of the heart to become abnormally thickened.
- Heart pumping strength. An echocardiogram can assist in determining the heart's pumping strength. Specific measurements may include a percentage of blood evacuated from a filled ventricle during each heartbeat (ejection fraction) or a volume of blood pumped by the heart in one minute (cardiac output).
- Damage to the heart muscle. During an echocardiogram, it is possible to determine whether all parts of the heart wall are contributing normally to heart pumping activity. Parts that exhibit weak movement may have been damaged during a heart attack or be receiving too little oxygen. This may indicate coronary artery disease or various other conditions.
- Valve problems. An echocardiogram indicates movement of heart valves as the heart beats. It can be determined from this if the valves open sufficiently wide for adequate blood flow (i.e. no stenosis) and/or close fully for preventing blood leakage (i.e. no valve regurgitation).
- Heart defects. Many heart defects may be detected with an echocardiogram, including problems with heart chambers, abnormal connections between the heart and major blood vessels, and complex heart defects that may be present at birth. Echocardiograms can also be used to monitor a baby's heart development before birth.

In addition to the above, it is also possible (using more advanced analysis techniques) to assess heart wall thickness, wall kinetics and blood flow patterns.

Various different hardware implementations for ultrasound examinations exist.

The most common approach takes the form of an ultrasound probe having an array of ultrasound transducers acoustically coupled to a skin contact area located at its tip. This is slid across the patient's skin, typically using acoustic coupling gel applied between the skin and the probe. The ultrasound probe may be a handheld probe device connected to an ultrasound imaging system or device, for instance mounted in the form of a cart or trolley.

Ultrasound probes utilize ultrasound transducers to generate the acoustic signals. Different types of ultrasound transducer exist. The most common type of transducer are piezoelectric transducers.

One alternative, and advantageous, type are capacitive micromachined ultrasonic transducers (CMUT). CMUT transducers are a relatively recent development. CMUTs utilize changes in capacitance for providing the energy transduction function. CMUTs are constructed on silicon using micromachining techniques. A cavity is formed in a silicon substrate, and a thin membrane suspended over the cavity on which a metallized layer acts as an electrode. The silicon substrate serves as a lower electrode.

As CMUTs are micromachined devices, it is simpler using this technology to construct 2D and 3D arrays of transducers. This means large numbers of CMUTs can be included in a transducer array, providing larger bandwidth compared to other transducer technologies.

Furthermore, achieving high frequency operation is also easier using CMUTs due to their smaller dimensions. The frequency of operation depends upon the transducer cell size (in particular, the size of the cavity covered by the membrane), and also upon the stiffness of the material used for the membrane.

Furthermore, as CMUT transducers are constructed on silicon, integration of additional control or driving electronics is also easier compared to other transducer technologies. This provides the potential for reducing form factor of a device by integrating control components on the same chip as the transducers for instance.

PMUT transducers may also be used in alternative examples.

An alternative hardware approach is use of an electronic stethoscope, which assists in performing cardiac auscultation. Recent developments have been made in this area for more sophisticated processing of auscultatory heart sound signals to enable for example improved analysis and clarification of the resulting sounds, for enabling diagnosis based on the results.

With advancements in communication technology and computerization, remote monitoring has become possible. Due to difficulty in bringing a patient to hospital or due to non-availability of doctors, on many occasions a remote monitoring of physical parameters is preferable. Additionally, hands-free autonomous monitoring in general is also beneficial, for instance in cases where a clinician requires cardiac information in parallel with performing another clinical operation or examination with the patient.

Electrocardiography (ECG or EKG) is the process of recording the electrical activity of the heart over a period of time using electrodes placed over the skin. These electrodes detect the small electrical changes on the skin that arise from the heart muscle's electrophysiological pattern of depolarizing and repolarizing during each heartbeat.

In a conventional 12-lead ECG, ten electrodes are placed on the patient's limbs and on the surface of the chest. The overall magnitude of the heart's electrical potential is then measured from twelve different angles (otherwise referred to as "leads") and is recorded over a period of time (typically ten seconds for instance). In this way, the overall magnitude and direction of the heart's electrical depolarization is captured at each moment throughout the cardiac cycle. The graph of voltage versus time produced by this noninvasive medical procedure is an electrocardiogram.

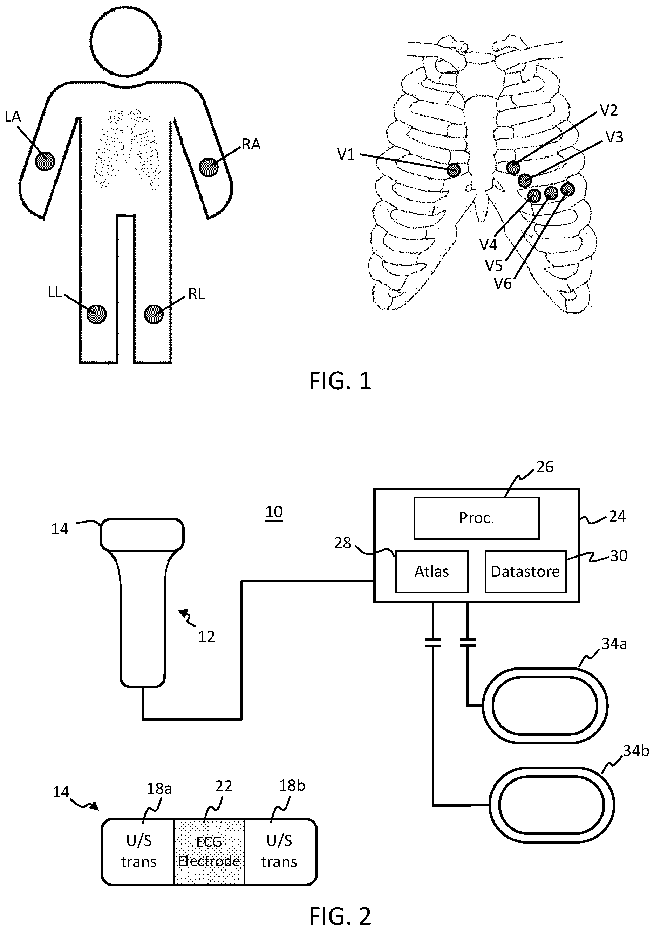

The ten standard electrode positions are set out in Table 1 below, and illustrated schematically in Fig. 1.

**Table 1**

| Electrode Position label | Electrode Position |
|---|---|
| LA | Left arm |
| RA | Right Arm |
| LL | Left leg |
| RL | Right leg |
| V1 | 4th intercostal space, right of sternum |
| V2 | 4th intercostal space, left of sternum |
| V3 | between V2 and V4 |
| V4 | 5th intercostal space, at the midclavicular line |
| V5 | same horizontal level as V4, but on the anterior axillary line |
| V6 | same horizontal level as V4, but at the midline |

The standard twelve ECG leads have the following standard labels: I, II, III, aVR, aVL, aVF, V1, V2, V3, V4, V5, V6, and are categorized into three categories: limb leads, augmented limb leads and precordial leads.

Each of the different ECG lead measurements are computed using a combination of either two or three of the electrodes. Table 2 below sets out how each of the standard twelve leads is calculated.

**Table 2**

| ECG leads = measured potential from electrodes | | | |
|---|---|---|---|
| **Limb leads** | **Augmented limb leads** | **Precordial leads** | |
| I = LA-RA | aVR=RA-(LA+LL)/2 | V1=V1-(RA+LA+LL)/3 | V4=V4-(RA+LA+LL)/3 |
| II = LL-RA | aVL=LA-(RA+LL)/2 | V2=V2-(RA+LA+LL)/3 | V5=V5-(RA+LA+LL)/3 |
| III = LL-LA | aVF=LL-(RA+LA)/2 | V3=V3-(RA+LA+LL)/3 | V6=V6-(RA+LA+LL)/3 |

The electrode at the right leg (RL) is not used in the leads, and is only used as ground reference. The voltage of a virtual point called the Wilson Central Terminal (WCT) is subtracted from each of the precordial electrode potentials. The WCT is obtained by averaging the potential at the limbs (WCT=(RA+LA+LL)/3) referred to the reference electrode on the right leg.

At any given instant during the cardiac cycle, all ECG leads analyze the same electrical events but from different angles. This means that ECG leads with similar angles must display similar ECG curves (diagrams). For some purposes (e.g. diagnoses of arrhythmias) it is not always necessary to analyze all leads, as the diagnosis can often be established by examining fewer leads. On the other hand, for the purpose of diagnosing morphological changes (e.g. myocardial ischemia), the more leads that are used, the more accurate the diagnosis. Thus, depending upon the pathology being investigated, different numbers of leads may be needed.

The 12-lead ECG is a trade-off between sensitivity, specificity and practicality. For example, in an extreme case, 120 leads (which has been tested in several studies on acute myocardial infarction) would improve sensitivity for many conditions, at the expense of specificity and practicality. On the other hand, using only one lead would allow for diagnosing some arrhythmias, but not all, and it would not allow diagnosis of morphological changes in the heart.

A multi-lead ECG would be of significant benefit as a supplement to the obj ective physical examination of the heart. However, the general physician typically does not have the equipment, time or experience needed to accurately and precisely place the required set of twelve leads at the correct locations on the body, in order thereby to carry out such a multi-lead ECG examination (this including positioning the electrodes, selecting the leads, reading the ECG results).

An improved ECG measurement approach would therefore be of value.

### SUMMARY OF THE INVENTION

The invention is defined by the claims.

According to examples in accordance with an aspect of the invention, there is provided a medical sensing system, comprising:
a probe unit, the probe unit comprising an ultrasound sensing means, and at least one electrode for use in electrocardiogram, ECG, measurement;
a controller operably coupled with the probe, and the controller comprising an atlas dataset storing reference ultrasound data associated with each of a plurality of possible locations of the probe relative to the body (e.g. relative to the heart), and comprising a datastore storing a plurality of reference (target) electrode locations relative to the body for different ECG measurements,
the controller being configured in at least one operation mode to control the ultrasound sensing means to acquire ultrasound data, and to implement a position guidance function configured to generate guidance information for a user for positioning the probe in one or more of the reference electrode locations based on the acquired ultrasound data, and based on reference to the atlas dataset.

Instead of a using a plurality of electrodes for an ECG examination, embodiments of the present invention propose use of a single mobile ECG electrode, integrated into a probe unit, which is moveable between different locations on the body. This way a multi-leads ECG examination can be performed, but without the need to position a large number of different electrodes on the subject's body.

At least one reference static electrode may additionally be provided, mounted to a fixed point on the user's body, for electrically coupling with the moveable electrode to perform examinations. The moveable ECG electrode can be thus moved between different standard ECG electrode locations, for acquiring ECG data for different of the standard angles (leads) discussed above which are needed for a full ECG examination (e.g. 9 or 12 lead examination). Thus, in effect, the system replaces a plurality of ECG leads (and electrodes) for placement at a set of different locations, with a single ECG electrode sequentially moveable between the different locations. When used in operation, this is combined with one or more static electrodes (which may be part of the provided system, or may be auxiliary electrodes, provided separately) which electrically coupled with the moveable probe electrode to enable ECG measurement for the different leads.

As discussed above, correct positioning of the ECG electrodes for the different standard angles is difficult for a non-expert. Thus, embodiments of the present invention further propose integration of an ultrasound sensing means (e.g. transducer arrangement) into the same moveable probe housing the ECG electrode, and wherein the acquired ultrasound data can be used to guide positioning of the ECG electrode (i.e. of the probe). An atlas dataset is stored which associates reference ultrasound data with different body locations, and a datastore is referred to which stores standard locations for ECG electrode placement for performing a standard ECG examination, e.g. 9 or 12 lead examination. Using a combination of the atlas and the datastore of reference locations, the system can guide the probe to each of a set of one or more target locations (e.g. through a sequence of standard locations).

The system may control acquisition of ECG measurement data once the probe is located in each given reference target location.

Thus, a sequential or step-wise ECG examination system is provided which can be operated even by a non-expert by virtue of an ultrasound-based position guidance function for guiding accurate placement of the probe.

The controller may generate an information output indicative of or based on the guidance information, i.e. a guidance output.

The guidance information may for example include graphical data for display on an associated display device, which may or may not be included as part of the system. For example, the graphical output may provide a visual indication as to a direction and/or distance in which to move the probe in order to arrive at the target electrode location. The guidance information output may additionally or alternatively comprise one or more other sensory outputs, such as auditory information and/or haptic feedback for the user. Haptic feedback might be provided via a handle of the probe unit for instance.

The ultrasound sensing means comprises for example an ultrasound transducer arrangement, i.e. it comprises one or more ultrasound transducers. It may comprise an ultrasound transducer array.

The reference ultrasound data may for example reference ultrasound imaging data.

The atlas referred to above simply means a dataset comprising reference ultrasound data associated with each of a plurality of possible locations of the probe, e.g. data as acquired by a probe which is positioned in each of those locations. The term 'atlas' may simply be understood as referring to a dataset therefore, and may be otherwise referred to as an atlas dataset.

The guidance function may be configured to generate guidance information for guiding the user to position the probe in a pre-defined set of reference electrode locations, for acquiring a pre-defined set of ECG measurements.

In other words, in this example, the controller guides the user through a plurality of reference electrode positions relative to the body (in particular, relative to the heart). Each location is for collecting ECG measurements corresponding to a different angle relative to the heart (i.e. a different a 'lead').

The set of reference electrode locations may have a defined order, so that they define a sequence of locations. In this way, the user may be guided through a sequence or series of reference electrode locations in a particular order. For example, this may be based on ordering the reference positions so as to minimize travel distance between reference electrode positions. However, a pre-defined order is not essential.

Reference electrode positions may be chronologically grouped in some examples to minimize travel distance between reference positions of a common group.

The controller is preferably further configured to control acquisition of ECG measurement data using the at least one ECG electrode of the probe when the probe is positioned in each of the one or more target locations, i.e. the controller implements an ECG measurement in each of the one or more target locations.

This may comprise controlling electrical stimulation between the at least one ECG electrode integrated in the probe and at least one further reference electrode. The reference electrode may for instance be a static reference electrode, provided mounted to the subject's body at a suitable reference location (e.g. the left or right leg, or left or right arm). The one or more reference electrodes pair with the moveable electrode in the probe to perform the ECG measurements.

The controller may automatically trigger acquisition of ECG data responsive to the probe reaching the target locations, or acquisition might be triggered manually by a user, for instance via a user interface or by actuating a user control (e.g. button) on the probe unit.

The controller may be further configured to determine one or more anatomical parameters pertaining to the heart based on acquired ECG data for each of the one or more target locations.

The system may further include at least one static ECG electrode for statically mounting to a location on the body of the subject, for electrically coupling with the at least one ECG electrode of the probe, for performing ECG measurements.

The at least one static electrode is separate from the probe and is intended to be spatially displaced from the probe during use, for acquiring ECG measurements using the probe electrode and the static electrode.

For example, the one or more static electrodes might be mounted in one or more of the standard electrode locations for a twelve or nine lead ECG examination (see Table 1 above), e.g. the left leg, right leg, left arm, right arm, etc.

In advantageous examples, the at least one static ECG electrode may be integrated in a body-wearable unit.

A wearable unit means a unit which can be worn by the user on a part of their body, e.g. a wrist, ankle, foot, leg or arm for instance. It may comprise a band, for instance which can be fitted around an ankle, arm, wrist or leg.

The advantage of a wearable unit is that it ensures a fairly reliable positioning of the sensor relative to the user's body. Since the wearable unit mounts to a known body location, with known typical shape and contours, the electrode placement within the unit can be configured such that, when the unit is worn, the electrode is placed in a reliably known location on the body, potentially improving ECG measurement accuracy.

According to one or more examples, the position guidance function may be based at least in part on acquiring ultrasound data representative of an area containing at least a portion of the ribs of the patient, and wherein the atlas dataset comprises reference ultrasound data pertaining to the ribs for different locations. This ultrasound data may thus be collected based on acquiring data (e.g. M-mode data) at a relatively shallow level in the patient's chest. This may be controlled by a beamformer included in the system for example.

Additionally or alternatively, the position guidance function may be based at least in part on collecting ultrasound data capturing a region containing at least a portion of the heart. It may be based on use of an image registration procedure to determine a position offset of the probe with respect to one or more of the target ECG electrode locations. To capture the heart, ultrasound data from a slightly lower depth within the chest may be captured. The image registration option will be further discussed later.

In accordance with one or more examples, the acquired ultrasound data may include ultrasound image data, and wherein the system stores a reference ultrasound image view associated with each target ECG electrode location, and wherein the image registration procedure comprises performing registration between acquired ultrasound image data and a reference ultrasound image view for a given target location (i.e. for the target ECG electrode location to which the probe is being guided).

For example, if the reference image is not in registration with the captured image view at the given probe location, this means that the probe needs to be moved in order to arrive at the target electrode location. By determining an offset between the two image views, a distance that the probe needs to be shifted may be determined and provided as part of the guidance feedback for example.

The reference image views for the different locations may be stored by the atlas dataset or the datastore, or by a different storage component.

In accordance with one or more embodiments, the position guidance function may have two modes:
a first mode in which the ultrasound sensing means is controlled to acquire ultrasound data at a first depth level in the body, for acquiring data representative of at least a region of the ribs of the subject, and wherein the position guidance is based on atlas data pertaining to rib locations; and
a second mode in which the ultrasound sensing means is controlled to acquire ultrasound data at a second, deeper, depth level in the body, for acquiring ultrasound data representative of the heart, and preferably wherein the position guidance is based on an image registration procedure, based on atlas data pertaining to the heart.

The first mode may be operated when the probe is far away from the target location, and the second when the probe is closer.

The first mode is for example a broad positioning mode, for moving the probe to the general vicinity or area of the target location. The second mode is for example a fine tuning mode, for fine adjustment of the probe location.

In some examples, in the first mode, the position guidance information may comprise an indication of at least a direction in which the probe should be moved to arrive at the target location, and wherein in the second mode the position guidance information comprises an indication of a direction and distance in which the probe should be moved to arrive at the target location.

In some examples, the controller may be configured to recurrently perform a mode selection procedure, wherein
the position guidance function operates in the second mode if at least a portion of the reference ultrasound image view for the given target location is within the field of view of the acquired ultrasound data; and
the guidance function operates in the first mode if no part of the reference ultrasound image view for the target location in question is found within the field of view of the acquired ultrasound data.

According to one or more advantageous embodiments, the system may be configured to guide the user to position the probe in a first set of target electrode positions for performing a first set of one or more ECG measurements, and to adjust the stored target electrode locations for a second set of one or more ECG measurements based on ECG measurement data acquired in the first set of ECG measurements.

For example, based on the first one or more ECG measurements, it may be determined that the size, position, and/or angular orientation of the heart is different from that which is standard, or that which the atlas data and/or the reference location are based on. Thus, the atlas data and/or the reference locations for the remaining ECG measurements can be adjusted so that position guidance is tailored or personalized to the specific anatomy of the patient in question.

The position guidance information may comprise an indication of a suggested movement direction for the probe, for moving toward the target location.

Optionally, the position guidance may further comprise an indication of a movement distance.

In one or more advantageous embodiments, the controller may be configured in at least one operation mode to acquire ultrasound data continuously or at regular intervals, and wherein the position guidance function comprises a continuous or recurrent feedback loop between acquired ultrasound data and the generating of the position guidance information.

For example, ultrasound data may be continuously acquired at a first (shallow) depth level within the subject's body, for instance at the level of the subject's ribs.

The controller can continually check the updated positioning of the probe relative to the target location and continually update the guidance information accordingly.

Examples in accordance with a further aspect of the invention provide a method for guiding a user in positioning a medical sensing probe relative to a subject's body, the probe comprising an ultrasound sensing means and at least one ECG electrode, and the method comprising:
acquiring ultrasound data using the ultrasound sensing means;
accessing an atlas dataset storing reference ultrasound data associated with each of a plurality of possible locations of the probe relative to the body;
accessing a dataset storing a plurality of reference or target electrode locations relative to the body for different ECG measurements, and
implementing a position guidance function configured to generate guidance information for a user for positioning the probe to one or more of the reference electrode locations based on the acquired ultrasound data, and based on reference to the atlas dataset.

The guidance function is in advantageous examples configured to generated guidance information for guiding the user to position the probe through a pre-defined set of reference electrode locations, for acquiring a pre-defined set of ECG measurements. The set of reference electrode locations may have a predefined order in some examples, such that they define a predefined sequence of locations.

Examples in accordance with a further aspect of the invention provide a computer program product comprising code means, configured when run on a processor, the processor being communicatively coupled with a probe comprising an ultrasound sensing means and at least one ECG electrode, to cause the processor to perform a method in accordance with any example or embodiment outlined above or described below, or in accordance with any claim of this application

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 schematically illustrates standard electrode locations for a standard twelve lead ECG examination;
Fig. 2 shows an example system in accordance with one or more embodiments;
Fig. 3 shows an example probe unit of an embodiment in use;
Fig. 4 shows example alternative configurations for the ultrasound sensor means and ECG electrode in the head of a probe unit;
Fig. 5 shows locations for the moveable probe electrode and a static reference electrode for performing a twelve lead ECG examination according to one or more embodiments;
Fig. 6 outlines in block diagram form an example workflow for performing a sequential or step-wise ECG examination in accordance with one or more embodiments;
Fig. 7 shows in block diagram form an example mode selection procedure for use according to one or more examples of the position guidance function;
Fig. 8 illustrates an example visual output of results of an ECG examination according to one or more examples;
Fig. 9 shows locations for the moveable probe electrode and a static reference electrode for performing a nine lead ECG examination according to one or more embodiments; and
Fig. 10 outlines components of an example ultrasound imaging system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system for performing ECG measurements and comprises a probe with an integrated one or more ECG electrodes and an ultrasound sensing means, such as a transducer arrangement. The probe thus provides a mobile ECG electrode which can be sequentially moved between a set of different locations on the body for acquiring ECG measurements from different angles relative to the heart (i.e. different 'leads'). Positioning of the probe in each required location is guided by a position guidance function which uses ultrasound data acquired by the ultrasound sensing means to locate the probe (with reference to an ultrasound body atlas or map), and uses a stored set of reference body locations to then guide a user with guidance information as to how to move the probe to arrive at a next target electrode location. In examples, the user may be guided through a sequence of electrode locations, with ECG data acquired at each one, thereby sequentially building up a set of standard ECG lead measurements.

Thus embodiments of the invention propose to assemble a multiple-lead ECG measurement by sequential single-lead ECG measurements.

The main elements of the system include an ultrasound imaging system and an integrated single electrode (or electrode arrangement) and ultrasound transducer probe. ECG positioning guidance is provided, aided by location recognition via collected ultrasound data, e.g. ultrasound imaging. One or more algorithms may be included for determining a next probe point in for instance a sequence of target ECG measurements. In some examples, a data processing algorithm may further be included for assembling a multi-lead ECG examination report or output result from a set of acquired single-lead ECG measurements.

An exploring (e.g. positive) ECG electrode is integrated in the ultrasound probe, for instance such that ultrasound transducer arrangement or array can image an area around the electrode location. Depending upon which ECG measurements (leads) need to be acquired, one or more reference (e.g. negative) ECG electrodes may be provided for mounting in a static location of the user's body, e.g. arms and/or legs, e.g. by integrating them in wrist or ankle bands for instance.

An example medical sensing system in accordance with one or more embodiments is schematically shown in Fig. 2.

The system 10 comprises a probe unit 12. A head 14 of the probe unit is shown from a front-facing view at the bottom of Fig. 2. The probe unit comprises an ultrasound (US) sensing means 18, and at least one electrode 22 for use in electrocardiogram, ECG, measurement. The ultrasound sensing means in this example comprises an arrangement of one or more ultrasound transducers (for example CMUT or PMUT transducers), for instance an ultrasound transducer array. The ultrasound sensing means may acquire for example ultrasound image data.

In the example shown in Fig. 1, the ultrasound sensing means 18 comprises a first 18a and second 18b portion, arranged either side of the ECG electrode. However, in other examples, the sensing means may comprise only a single part, or more than two parts for instance.

The system further comprises a controller 24 operably coupled with the probe unit 12, and the controller comprising an atlas dataset 28 storing reference ultrasound data associated with each of a plurality of possible locations of the probe 12 relative to a subject's body (e.g. heart), and comprising a datastore 30 storing a plurality of target/reference electrode locations (relative to the body) for different ECG measurements. Although the atlas dataset and datastore are shown as separate components in Fig. 1, they may be incorporated in a single data storage element in other examples.

The controller in the illustrated example further comprises a processing component 26 for performing control and processing operations, and this being in communication with the atlas dataset 28 and the datastore 30. In further examples, the atlas and datastore and processor may all be integrated in a single controller or processor component, instead of separate components.

The controller 24 (e.g. processor element 26) is configured in at least one operation mode to control the ultrasound sensing means 18 to acquire ultrasound data, and to implement a position guidance function configured to generate guidance information for a user for positioning the probe 12 in one or more of the reference electrode locations (stored in the datastore 30) based on the acquired ultrasound data, and based on reference to the atlas dataset 28.

The datastore may for example comprise a database of different ECG electrode locations required for each of a set of standard ECG examinations, e.g. 9-lead, 12-lead, etc.

The atlas dataset effectively provides an ultrasound map, linking sample acquired ultrasound data with a corresponding location on the body. For instance it may store for each possible location of the probe on the body an ultrasound imaging view which is observed by the probe when at that location. Thus, by querying or searching the atlas with acquired ultrasound data at a given probe location (during use), the system can determine in which location the probe is currently positioned.

Although a single controller 24 is referred to above, the controller may comprise a plurality of controller components (e.g. a plurality of processors), or may comprise a single controller component (e.g. a single processor). The functions attributed to the controller in this disclosure may thus be distributed between one or more processing components in different examples.

The system preferably further includes at least one static ECG electrode 34 for statically mounting to a location on the body of the subject, for electrically coupling with the at least one ECG electrode of the probe unit 12, for performing ECG measurements. Alternatively, in use, one or more auxiliary static electrodes (not part of the provided system) may be used for electrically coupling with the at least one probe electrode for performing ECG measurements.

In the example of Fig. 2, two static reference electrodes 34a, 34b are shown, in the form of wrist and/or ankle mountable straps or bands 34a and 34b.

In alternative examples, the one or more static sensors may be integrated in a different unit, for instance a different types of body-wearable unit, or a unit for mounting to the body in a different way such an adhesive patch or clip.

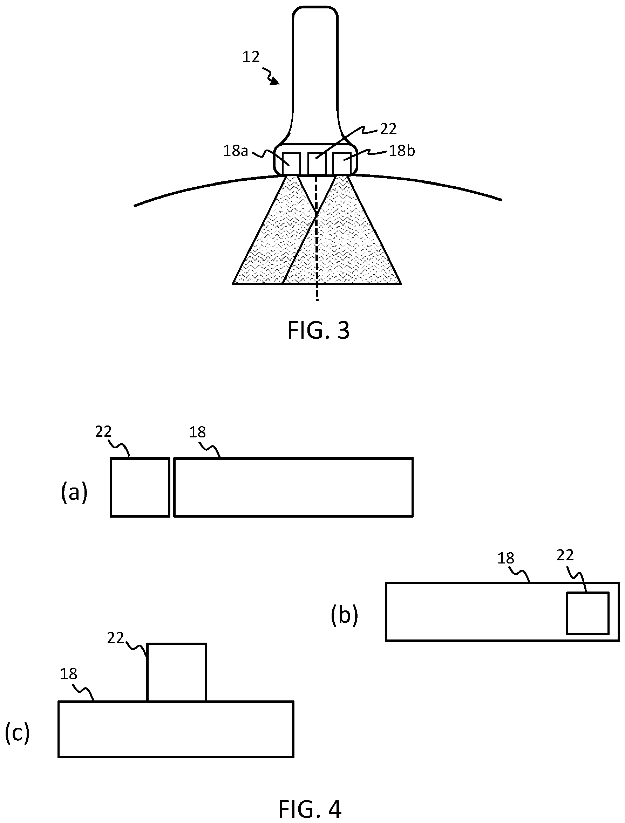

Fig. 3 schematically illustrates the probe 12 of the system in use, in position on a subject's chest, and with the first 18a and second 18b ultrasound transducer arrangements transmitting ultrasound waves, and the ECG electrode 22 performing ECG sensing.

In the example of Fig. 1, the ECG electrode 22 is show as positioned symmetrically between two ultrasound transducer arrangements 18a, 18b. This has the advantage that the position of the ECG probe is independent of the in-plane angle at which the probe is positioned on the patient.

However, in other examples, a single ECG electrode 22 may instead be provided positioned adjacent to a single ultrasound transducer 18. This alternative arrangement is illustrated in Fig. 4, labelled (a). Such a system would allow for a larger US transducer in the same probe unit 12 footprint. This may result in improved imaging quality compared to a single smaller transducer, or even two smaller transducers) and may be of lower cost (due to reduced driving circuitry and driving processors). In such a system the positioning algorithm may need to take into account the angle of the US guidance image in order to correctly position the probe.

Fig. 4 shows two further alternative arrangements for the ultrasound sensing means 18 and the ECG electrode 22. In example (b), the ECG electrode is positioned within the footprint of the ultrasound transducer arrangement 18 itself. In example (c), the ECG electrode is provided displaced perpendicular to the longitudinal axis of the transducer arrangement, i.e. above the transducer arrangement. Any other relative configuration of the ultrasound sensing and ECG electrode components is also possible.

As discussed above, in preferred embodiments, the position guidance function of the controller 24 is configured to generate guidance information for guiding the user to position the probe in a pre-defined set of reference electrode locations, for acquiring a pre-defined set of ECG measurements.

In other words, the controller guides the user through a series of reference electrode positions relative to the body (in particular, relative to the heart). Each location is for example for collecting ECG measurements corresponding to a different angle relative to the heart (i.e. a different lead of a multi-lead examination).

The pre-defined set of reference electrodes may have a pre-defined order in some examples, such that they define a pre-defined sequence of reference locations, however, this is not essential.

The controller is preferably configured to control acquisition of ECG measurement data using the at least one ECG electrode 22, when the probe 12 is positioned in each of the one or more target locations, i.e. the controller implements an ECG measurement in each in of the one or more target locations.

This may comprise controlling electrical stimulation between the at least one ECG electrode 22 integrated in the probe 12 and the at least one further reference electrode 34. For the example of Fig. 2 for instance, static reference electrodes 34a, 34b are used. One or both of these may be used depending upon the ECG measurement being collected.

The controller 24 may be configured to automatically trigger acquisition of ECG data responsive to the probe unit 12 reaching a given target location, or acquisition might be triggered manually by a user, for instance via a user interface or by actuating a user control (e.g. button) on the probe unit 12.

The controller may further be configured to determine one or more anatomical parameters pertaining to the heart based on acquired ECG data for each of the one or more target locations, e.g. heart size, heart location, heart orientation, thickness of one or more heart walls, size of one or more heart chambers or any other parameter that is typically derivable from ECG measurement data.

In accordance with one or more embodiments, the controller is configured in at least one mode to guide a user through the appropriate sequence of electrode locations for performing a standard 12 lead ECG examination. The required positions of the probe unit 12 for performing each of the standard 12 lead measurements may be stored in the reference datastore 33, and wherein the controller 23 uses the atlas dataset 28 to navigate the user in positioning the probe at each of the locations, based on for instance continually or recurrently collected ultrasound data.

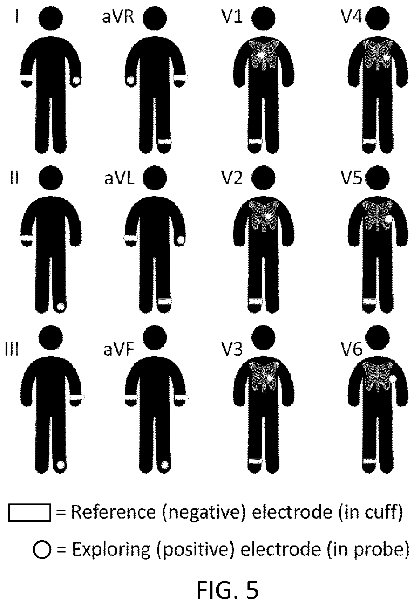

Thus, to obtain the ECG measurement data for a standard 12-lead ECG, the controller guides the user through a sequence of 12 single-lead ECG measurements, for each one of which the probe electrodes 22 and the static reference electrode(s) 34a, 34b, need to be placed in the standard locations as indicated in Table 3 below, and illustrated schematically in Fig. 5

**Table 3**

| ECG Lead Measurement | Probe ECG electrode (Exploring Electrode (+)) | Static reference electrode 1 (-) | Static reference electrode 2 (-) |
|---|---|---|---|
| I | Left arm (LA) | Right arm (RA) | |
| II | Left leg (LL) | Right arm (RA) | |
| III | Left leg (LL) | Left arm (LA) | |
| aVR | Right arm (RA) | Left arm (LA) | Left leg (LL) |
| aVL | Left arm (LA) | Right arm (RA) | Left leg (LL) |
| aVF | Left leg (LL) | Right arm (RA) | Left arm (LA) |
| V1 | 4th intercostal space, right of sternum | Right leg (RL) | |
| V2 | 4th intercostal space, left of sternum | Right leg (RL) | |
| V3 | between V2 and V4 | Right leg (RL) | |
| V4 | 5th intercostal space, at the midclavicular line | Right leg (RL) | |
| V5 | same horizontal level as V4, but on the anterior axillary line | Right leg (RL) | |
| V6 | same horizontal level as V4, but at the midline | Right leg (RL) | |

For more detailed reference, the locations on the body of the different electrode positions are shown in Fig. 1 (discussed above).

Fig. 5 schematically illustrates the moveable 22 and static 34a, 34b ECG electrode locations for each of the twelve standard ECG examination locations outlined in Table 3 above.

Placement of the static electrodes in the correct location on the arms and legs for each ECG lead measurement should be straightforward. In some cases, the static electrodes can be left in place between measurements, but in other cases, they may need to be moved between measurements. Placement can be made particularly straightforward in the case that the static electrodes are integrated in wearable units, such as wrist/ankle bands, as illustrated in Fig. 2. The controller may generate a prompt or other guidance for the user for each new measurement as to where the static ECG electrodes 34a, 34b need to be placed.

Placing the probe 12 with the exploring (positive terminal) electrode 22 in the correct positions for each of the measurements may be difficult for non-experts.

Thus, as discussed, the invention provides a position guidance function configured to use ultrasound data to locate the probe and to guide positioning of the probe to the reference locations of the ECG examination.

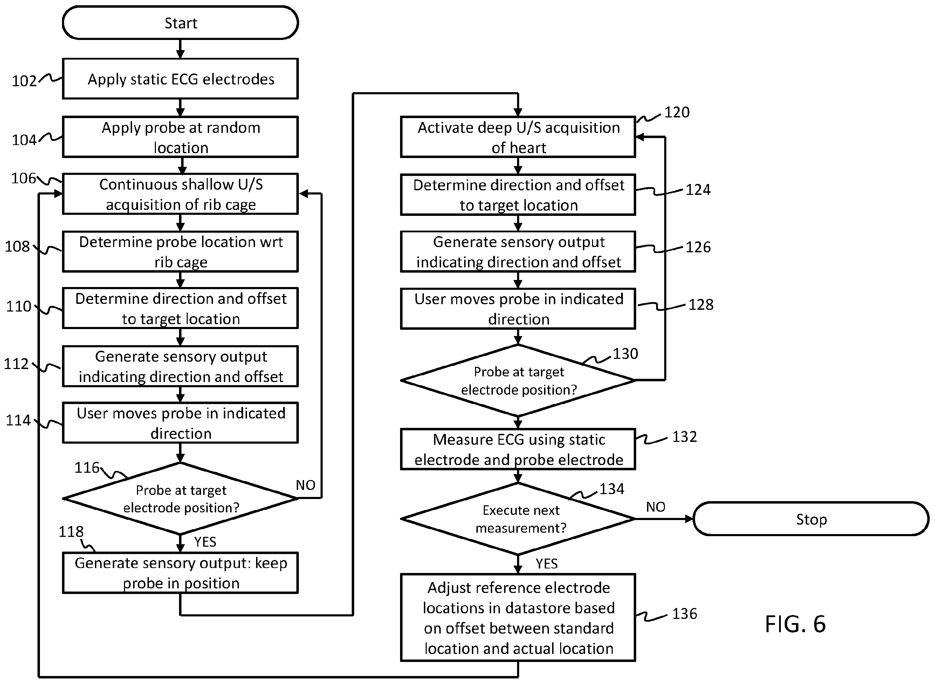

To further explain and aid understanding of the invention, steps of one example workflow for performing a sequential ECG examination using a system in accordance with one or more embodiments of the present invention are outlined in block diagram form in Fig. 6. The workflow includes both steps performed by a user during the procedure and those performed by the system (e.g. controller) during the procedure.

In a first step 102, the static ECG reference (negative) electrode(s) 34a, 34b are attached to the subject's limb(s) in the appropriate places (see e.g. Table 3 above), e.g. with ECG reference electrode(s) integrated in cuff(s) as discussed above.

In step 104, the probe unit with integrated ultrasound transducer 18 and ECG exploration (positive) electrode 22 is positioned on the patient's chest at any location.

In step 106, the system starts a continuous shallow ultrasound measurement to image the patient's rib cage. In other words, the system acquires ultrasound data at a first depth level in the body, suitable for capturing image data containing at least a portion of the ribs of the subject.

In step 108, the system uses stored ultrasound image data for the rib cage, stored in the atlas dataset 28, and applies a mapping algorithm to determine where the probe is located in a coordinate system with respect to the rib cage.

In step 110, the system calculates the direction and offset of the current position with respect to the target ECG electrode position for the first measurement. The reference datastore 30 for example stores coordinates for each of the target electrode positions, for instance relative to a reference frame of the rib cage.

In step 112, the system outputs guidance information, e.g. in the form of a sensory output, indicating to the user how far and in which the direction the probe 12 needs to be moved in order to arrive at the standard ECG electrode position.

Responsive to the guidance information, the user moves 114 the probe 12 into the indicated direction, while the system checks in step 116, whether the probe is yet in the target position. While the probe is not in the target position (it is still being moved by the user), the system repeats steps 106-116 in a loop, continuously re-acquiring ultrasound data, identifying the probe position, updating the guidance information and re-checking whether the target location has been reached.

Once the system detects 116 that the probe 12 has arrived at the relevant target ECG electrode position, the system generates sensory guidance output information indicating to the user to keep the probe in position.

The system then, in step 120, switches to a deep ultrasound measurement mode to image the patient's heart. In other words, the system changes mode to acquire ultrasound data at a second, deeper, depth in the subject's body, suitable for capturing at least a portion of the heart of the subject.

The personal anatomy of the subject, e.g. the exact location, size, orientation, of the heart, may mean that the standard (stored) ECG position with respect to the rib cage is not exactly correct for the patient, and an adjustment would improve the positioning. Thus, the system may perform an image alignment algorithm, in which captured image data of the heart at current probe location is compared with a stored reference ultrasound image view for the optimal target location relative to the heart, and in case of any offset, the user is guided to adjust the probe positioning.

The system may thus use the image alignment or registration algorithm to calculate 124 the direction and offset between the current position and the target position which provide the exact desired positioning with respect to the heart for capturing the ECG data (a 'personalized' ECG electrode position).

In step, 126, the system generates output guidance information (e.g. sensory output information) indicating to the user how far and in which the direction the probe needs to move (as a fine-tuning of the position) and attain the personalized ECG electrode position.

After the user has moved 128 the probe towards the personalized ECG electrode position, the system checks 130 whether the probe is at the target location, for instance by repeating the image alignment check mentioned above. If not, the system repeats steps 120-130 in a loop, re-acquiring deep ultrasound data, checking the image alignment, and updating the position guidance information if needed to guide the user toward the personalized target location.

Once the fine-tuned ECG electrode location has been reached, the system, in step 132, controls the ECG electrode 22 with required electrical stimulation signals for acquiring an ECG measurement at the current position. The measurement is done using the probe electrode 22 in combination with the appropriate static reference electrodes.

If there are more measurements to be acquired for the full ECG sequential examination (checked in step 134), the system then repeats steps 106 to 134 until all ECG measurements have been acquired.

Optionally, the system may also perform a procedure 136 to adjust the target locations stored in the reference datastore 30 for the different ECG electrodes, based on the patient's personal anatomy, as discovered during a previous one or more ECG measurements.

In particular, from the difference between the two (or more) target electrode positions (as stored in the datastore 30) and the corresponding actual (personalized) positions, as uncovered during fine adjustment steps 120-128, the system can adjust the remaining stored target locations for the particular patient, so that they are likely to better match the actual needed electrode positions for future ECG measurements.

The disparity between the stored (standard) ECG electrode locations and the actual (personalized) optimum ECG electrode location may arise due to a number of different anatomical factors pertaining to the subject, which may include for instance:
- How the size of the subject's heart compares to the reference size as expected from the measured rib geometry
- How the position of subject's heart compares to the reference position as expected from the measured rib geometry
- How the angular orientation of subject's heart compares to the reference angular orientation as expected from the measured rib geometry.

The adjusting of the target locations stored in the reference datastore 30 for the different ECG electrodes may be based on any one of more of these factors for example.

The adjustment might be done in any of a variety of different ways, e.g. through a simple scaling or through a more complex procedure. Applying this adjusted target location information in steps 108-114 for subsequent measurements may lead the user much more quickly to the precise (personalized) electrode position.

The adjustment procedure 136 may be applied even after just the first ECG measurement, which may then be used to improve the position guidance for the second measurement. As more measurements are collected, and the adjustment repeatedly performed, the mapping of personalized ECG positions will become increasingly more accurate.

Steps 106-118, in which shallow ultrasound data indicative of the ribs is collected and analyzed may be understood as a first mode of the position guidance function, and steps 120-132, in which deeper ultrasound data representative of the heart is captured may be understood as a second mode of the position guidance function. The first mode navigates the probe to vicinity of the target location, while the second mode effects a fine adjustment of the probe position relative to the heart.

A mode selection procedure might be performed in some examples by the system to determine which mode it should be operating in. This will now be explained below. The positioning guidance function of the system preferably uses a mapping model that can identify the current location from a local ultrasound image. Subsequently, the direction towards the target probe location is easily discernible from the vector between the current location and the target point. Depending upon how close the probe is to the stored reference target electrode location, either the first (shallow ultrasound) or second (deeper ultrasound) modes might be applied.

In some examples, the ultrasound sensing means 18 is configured to require ultrasound imaging data, and wherein the system 10 stores a reference ultrasound image view associated with each target ECG electrode location, and wherein an image registration procedure is performed which comprises performing registration between acquired ultrasound image data and a reference ultrasound image view for a given target location.

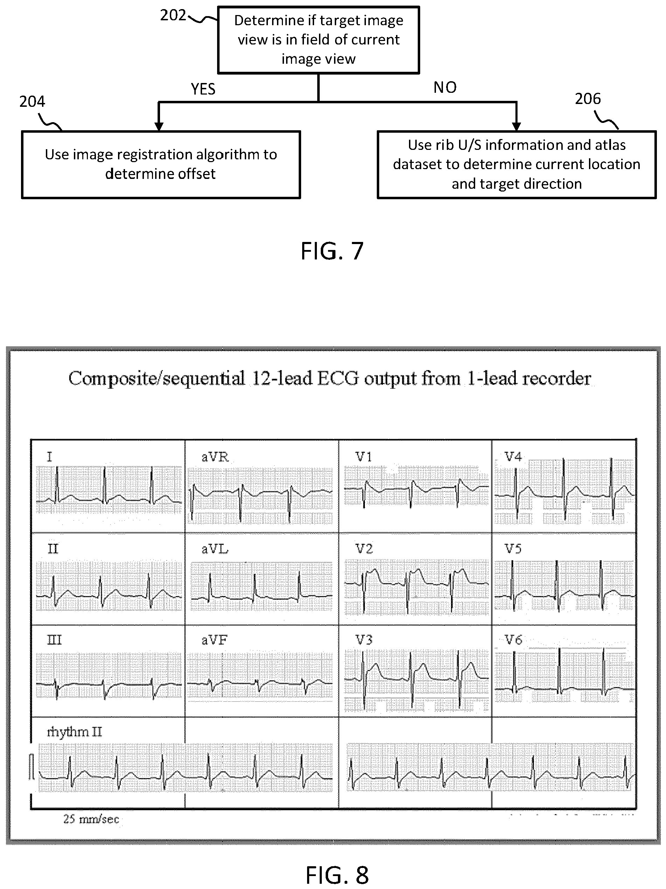

Fig. 7 outlines example workflow steps for the different modes.

In a first step 202, the system determines if the reference ultrasound image view for the given current location of the probe is within the field of view of the ultrasound image data acquired by the probe at the current location. An example may include to apply an image comparison or registration algorithm. A negative result between the required ultrasound image data for the current position and the reference ultrasound image view for the current position may indicate that the target location is outside of the field of view of the probe at its current location. Alternatively, a classifier algorithm (e.g. as part of a machine learning algorithm) be used to determine an anatomic similarity between the two views.

In the event that there is no overlap detected between the current probe ultrasound image view and the reference ultrasound image view for the target location, the system operates in the first mode discussed above (step 206 in Fig. 7). Here, the system acquires ultrasound image data at a shallow depth level within the body (for acquiring image data representative of the ribs of the subject) and uses reference data in the atlas dataset 28 to determine the current location of the probe and the target direction in which it should be moved to arrive at the target ECG electrode location. In particular, the system may search within the ultrasound atlas dataset 28, to find a match between the required ultrasound data at the current probe position and stored reference ultrasound data in the atlas dataset 28.

In an alternative example, a classifier algorithm might be employed, trained to predict from the current ultrasound image view the actual anatomic location. The deviation from the target ECG electrode location might be derived from a model estimate, e.g. calculated from the atlas. In this case, the distance to the target point is not of importance, but only the direction.

In the event that at least some overlap is detected between the ultrasound image view acquired at the current probe location and the reference ultrasound image view for the target electrode location, the system may operate in the second mode discussed above in which ultrasound data is acquired from a deeper depth level within the body of the subject and an image registration procedure performed between the acquired ultrasound image data of the heart and reference ultrasound image data to the target location pertaining to the heart. This is shown in step 204 of Fig. 7. Only relatively small adjustments are needed in this mode. The image registration algorithm can be used to determine an offset between the current image view and target image view (e.g. from a model database) to determine the exact deviation (distance + direction) to the target ECG electrode location.

This mode selection procedure may be applied within the workflow of Fig. 6 outlined above. For example it might be applied at step 108 where the system determines an initial probe location. Here it might be determined that the reference ultrasound image view is outside of the field of view of the current ultrasound image data. Hence the selection algorithm may proceed to step 206 of Fig. 7.

The mode selection procedure might also be applied for instance at step 134 of the workflow of Fig. 6. Here, since the probe is already close to the final target location, it is to be expected that the reference ultrasound image view is within the field of view of the acquired ultrasound image data. Hence the selection algorithm may proceed to step 204 of Fig. 7.

The position guidance information (output information) provided to the user may take the form of any kind of sensory output information, for example visual, audio, or haptic feedback. Haptic feedback might include vibration in the handle of the probe 12 for example, or in some examples might use a variable friction interface between the probe head 14 and the subject's body.

While ECG measurement data is accumulated, a standard 12-lead ECG report may be constructed using for example a suitable computation or accumulation algorithm. The results may be displayed on a display device, indicating the corresponding location of the different measurements, for example as illustrated in Fig. 8.

Examples discussed above have related to sequential or step-wise ECG examinations for acquiring a standard set of 12 ECG measurements (to simulate a twelve lead ECG examination). However, in further examples, the number of acquired ECG measurements can be different from twelve.

For example, according to one example embodiment, the system may be configured to guide the user through a set (or sequence) of nine ECG measurements.

Additionally, it is possible in this case to remove the need to adjust the positions of the static reference electrodes between different ECG measurements. For example, just the right ankle might be used as the reference electrode location for all measurements.

The procedure for acquiring the sequence of nine ECG measurements is substantially the same as that described above in relation to the example twelve-measurement acquisition procedure. However in this case, only one static reference electrode 34 is needed, for example positioned at the right leg of the subject, e.g. with the electrode incorporated in an ankle band.

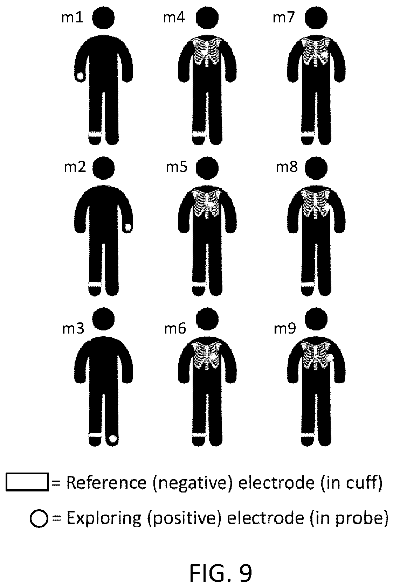

This may increase the efficiency of the measurement procedure workflow and may reduce the chance of errors or inaccuracies in the placement of the static ECG electrodes by the user. With the simplification of a constant reference electrode (e.g. at the right leg), the appropriate sequence of electrode configurations for a standard 12-lead ECG is reduced to nine measurements. The electrode location required for each of the nine ECG measurements according to this procedure is outlined in table 4 below and schematically illustrated in Fig. 9.

**Table 4**

| ECG Measurement | Probe electrode (+) location | Static reference electrode (-) location |
|---|---|---|
| m1 | Right Arm (RA) | Right Leg (RL) |
| m2 | Left Arm (LA) | |
| m3 | Left Leg (LL) | |
| m4 | V1; 4th intercostal space, right of sternum | |
| m5 | V2; 4th intercostal space, left of sternum | |
| m6 | V3; between V2 and V4 | |
| m7 | V4; 5th intercostal space, at the midclavicular line | |
| m8 | V5; same horizontal as V4, but at anterior axillary line | |
| m9 | V6; same horizontal as V4, but at the midline | |

In particular, Fig. 9 schematically illustrates the locations of the moveable (exploring) 22 and static (reference) 34 ECG electrode locations for each of the nine standard ECG examination locations outlined in Table 4 above. The reference electrode is schematically indicated by a white rectangle. The exploring (probe) electrode is schematically indicated by a white circle.

In either of the sequential ECG measurement procedures outlined above, the plurality of ECG measurements are calculated using the acquired ECG measurement data from each of the different probe positions. This employs use of a data processing algorithm. Such algorithms for computing the ECG measurement results from the acquired ECG measurement data from each of the 12 different electrode positions are well-known in the field.

By way of example, the twelve calculations required to be performed to determine the 12 ECG measurements for a standard 12 lead ECG procedure are outlined in Table 5 below.

**Table 5**

| ECG leads = required data-processing calculation | | | |
|---|---|---|---|
| Limb leads | Augmented limb leads | Precordial leads | |
| I = m2-m1 | aVR=m1-(m2+m3)/2 | V1=m4-(m1+m2+m3)/3 | V4=m7-(m1+m2+m3)/3 |
| II = m3-m1 | aVL=m2-(m1+m3)/2 | V2=m5-(m1+m2+m3)/3 | V5=m8-(m1+m2+m3)/3 |
| III = m3-m2 | aVF=m3-(m1+m2)/2 | V3=m6-(m1+m2+m3)/3 | V6=m9-(m1+m2+m3)/3 |

However, since in embodiments of the present invention, measurements are acquired stepwise, or sequentially, the data from the ECG electrode positions may not all be acquired from the same heart cycle. Due to beat-to-beat potential differences and heart rate variability, this can lead to the introduction of inaccuracy. To overcome this, the calculations may include an additional procedure for mapping the time and frequency between the different measurements (for registering the cardiac cycles of the different measurements to one another).

One possible approach to performing the required mapping (alignment) is for example to first detect the QRS complex of the heart cycle using ECG data, or just detect the R peaks from each of the ECG signals, and based on these reference points, the cardiac cycles between different of the measurements can be registered to one another. The detection of the QRS complex and/or R peak and or other characteristics of the ECG signal is a well-known procedure within the present field, and such registration procedure can work robustly even in the case of arrhythmias.

In various examples described above, guidance information is generated for guiding a user in moving the probe between different measurement positions. In further examples, a quick acquisition procedure may be implemented wherein the user moves the probe systematically across a large number of locations by sweeping it for instance across the chest surface systematically from top to bottom on both sides of the chest. For example, they might move the probe in a step-wise manner with regular spacing until the whole chest area has been covered. Ultrasound and ECG measurement signals may be acquired from all these locations. In this example, ultrasound and ECG measurement data are acquired from more measurement locations than are needed for compiling the final (e.g. 12 lead or 9 lead) ECG examination.

The system then selects from the total acquired dataset the data from just the measurement points that are needed for the particular ECG examination being compiled (e.g. 12 lead or 9 lead for instance). For instance, the datastore 30 may be consulted by the system to identify the plurality of target electrode locations relative to the body for the different target ECG measurements. The system may identify these locations from the total acquired ultrasound dataset based on the ultrasound data acquired from each of the multiple acquisition locations that the user scanned through. The system may for example compare the ultrasound data acquired from each of these locations with the reference ultrasound data stored in the atlas dataset 28 to determine the body location to which each of these scanned measurement points corresponds. The system may then select from these acquired measurement points the set of measurement points which correspond to the set of target electrode locations retrieved from the datastore 30 for the given measurement being compiled. The final ECG measurement may then be compiled using the ECG measurement data acquired from each of the selected set of the acquired measurement points.

The matching up of the acquired ultrasound data at each of the measurement points the user moved through with the target electrode locations stored in the datastore 30 may in some examples be performed based on use of a machine learning algorithm, for example a deep learning algorithm or model.

Hence, in this approach, measurement data is acquired from more locations than are needed, and the system uses the atlas dataset 28 and the datastore 30 to subsequently select the relevant ECG measurement data for the target electrode locations and compile the total ECG measurement (e.g. 9 lead or 12 lead) using these selected measurement points. This approach may lead to a quicker acquisition process for the user, which improves efficiency and convenience.

In a further possible development of this approach, the system may take into account the number of ECG measurements required for the full ECG examination being compiled. As discussed above, this can commonly be less than the full 12 lead ECG examination which is standardly used. In this example, guidance information may be generated for the user during the initial acquisition process which may indicate to the user when data from a sufficient number of ECG measurement points has been acquired. For example, the system may determine the body location of each acquired measurement point in real time, and keep track of from which of the target electrode locations the user has already acquired data, so that a guidance message can be communicated to the user when all of the required target electrode locations have been completed.

As discussed above, embodiments of the invention comprise acquisition of ultrasound data, for example ultrasound image data. In some examples, ultrasound images are acquired and analyzed as part of the position guidance function.

In some examples, an ultrasound imaging system may be provided, in association with the controller 24 of the medical sensing system 10 for controlling acquisition of ultrasound data using the ultrasound sensing means 18 of the probe unit 12 and/or generating one or more images from the data. The components of such a system may be integrated in the controller 24 or may be included in a separate ultrasound system to which the controller is operatively coupled in use.

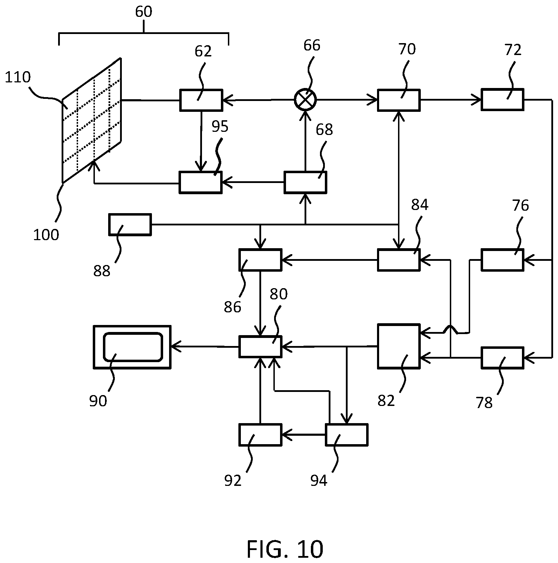

For reference, and by way of further, more detailed, explanation, the general operation of an exemplary ultrasound imaging system (suitable for application in accordance with one or more embodiments of the present invention) will now be described, with reference to Fig. 10.

The system comprises an array transducer probe 12 which has a transducer array 18 for transmitting ultrasound waves and receiving echo information. The transducer array 18 may comprise CMUT transducers; piezoelectric transducers, formed of materials such as PZT or PVDF; or any other suitable transducer technology. In this example, the transducer array 18 is a two-dimensional array of transducers 108 capable of scanning either a 2D plane or a three dimensional volume of a region of interest. In another example, the transducer array may be a 1D array.

The transducer array 18 is coupled to a microbeamformer 112 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is in general entirely optional. Further, the system includes a transmit/receive (T/R) switch 116, which the microbeamformer 112 can be coupled to and which switches the array between transmission and reception modes, and protects the main beamformer 120 from high energy transmit signals in the case where a microbeamformer is not used and the transducer array is operated directly by the main system beamformer. The transmission of ultrasound beams from the transducer array 18 is directed by a transducer controller 118 coupled to the microbeamformer by the T/R switch 116 and a main transmission beamformer (not shown), which can receive input from the user's operation of the user interface or control panel 138. The controller 118 can include transmission circuitry arranged to drive the transducer elements of the array 18 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 118 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 118 can be coupled to control a DC bias control 145 for the transducer array. The DC bias control 145 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 112 and are then passed to a main receive beamformer 120 where the partially beamformed signals from individual patches of transducers are combined into a fully beamformed signal, referred to as radio frequency (RF) data. The beamforming performed at each stage may be carried out as described above, or may include additional functions. For example, the main beamformer 120 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 122. The signal processor 122 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and microbubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Fig. 10 only the receiver beamformers 112, 120 are shown, for simplicity. In the complete system, there will also be a transmission chain with a transmission micro beamformer, and a main transmission beamformer.

The function of the micro beamformer 112 is to provide an initial combination of signals in order to decrease the number of analog signal paths. This is typically performed in the analog domain.

The final beamforming is done in the main beamformer 120 and is typically after digitization.

The transmission and reception channels use the same transducer array 18 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 126 and a Doppler processor 128. The B mode processor 126 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 132 and a multi-planar reformatter 144. The scan converter 132 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 140. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 142 converts the echo signals of a 3D data set into a projected 3D image as viewed from a given reference point as described in US Pat. 6,530,885 (Entrekin et al.).

The 2D or 3D images are coupled from the scan converter 132, multi-planar reformatter 144, and volume renderer 142 to an image processor 130 for further enhancement, buffering and temporary storage for optional display on an image display 140. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 128 and tissue structure information produced by the B mode processor 126 are coupled to a quantification processor 134. The quantification processor produces measures of different flow conditions such as the volume rate of blood flow in addition to structural measurements such as the sizes of organs and gestational age. The quantification processor may receive input from the user control panel 138, such as the point in the anatomy of an image where a measurement is to be made.

Output data from the quantification processor is coupled to a graphics processor 136 for the reproduction of measurement graphics and values with the image on the display 140, and for audio output from the display device 140. The graphics processor 136 can also generate graphic overlays for display with the ultrasound images. These graphic overlays can contain standard identifying information such as patient name, date and time of the image, imaging parameters, and the like. For these purposes the graphics processor receives input from the user interface 138, such as patient name. The user interface is also coupled to the transmit controller 18 to control the generation of ultrasound signals from the transducer array 6 and hence the images produced by the transducer array and the ultrasound system. The transmit control function of the controller 118 is only one of the functions performed. The controller 118 also takes account of the mode of operation (given by the user) and the corresponding required transmitter configuration and band-pass configuration in the receiver analog to digital converter. The controller 118 can be a state machine with fixed states.

The user interface is also coupled to the multi-planar reformatter 144 for selection and control of the planes of multiple multi-planar reformatted (MPR) images which may be used to perform quantified measures in the image field of the MPR images.

Advantageously, each of the B mode processor 126, Doppler processor 128, scan converter 132, multi-planar reformatter 144, volume renderer 142, image processor 130, quantification processor 134, and graphics processor 136 may be comprised by the control unit 24 of the system 10 in one or more embodiments of the present invention to facilitate image reconstruction and estimation of the physiological parameter of interest. In other examples, a separate ultrasound imaging unit may be provided comprising the components of the ultrasound imaging system outlined above, and configured for performing one or more of the discussed control and data processing operations.

Examples in accordance with a further aspect of the invention provide a computer program product comprising code means, configured when run on a processor, the processor being communicatively coupled with a probe comprising an ultrasound sensing means and at least one ECG electrode, to cause the processor to perform a method in accordance with any example embodiment outlined above or in accordance with any claim of this application.

As discussed above, embodiments make use of a controller 24 or control unit. The controller or control unit can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A medical sensing system (10), comprising:
a probe unit (12), the probe unit comprising an ultrasound sensing means (18), and at least one electrode (22) for use in electrocardiogram, ECG, measurement; and
a controller (24) operably coupled with the probe, and the controller comprising an atlas dataset (28) storing reference ultrasound data associated with each of a plurality of possible locations of the probe relative to the body, and comprising a datastore (30) storing a plurality of reference target electrode locations relative to the body for different ECG measurements,
the controller (24) being configured in at least one operation mode to control the ultrasound sensing means to acquire ultrasound data, and to implement a position guidance function configured to generate guidance information for a user for positioning the probe in one or more of the reference electrode locations based on the acquired ultrasound data, and based on reference to the atlas dataset.

2. A system (10) as claimed in claim 1, wherein the guidance function is configured to generate guidance information for guiding the user to position the probe in a predefined set of reference electrode locations, for acquiring a predefined set of ECG measurements.

3. A system (10) as claimed in claim 2, wherein the predefined set of reference electrode locations has a defined order, such that the guidance information is for guiding the user through a predefined sequence of reference electrode locations having a defined order.

4. A system (10) as claimed in any of claims 1 to 3, wherein the controller is further configured to control acquisition of ECG measurement data using the at least one ECG electrode of the probe, when the probe is positioned in each of the one or more target locations.

5. A system (10) as claimed in claim 4, wherein the controller is further configured to determine one or more anatomical parameters pertaining to the heart based on acquired ECG data for each of the one or more target locations.

6. A system (10) as claimed in any of claims 1-5, wherein the system further includes at least one static ECG electrode for statically mounting to a location on the body of the subject, for electrically coupling with the at least one ECG electrode of the probe, for performing ECG measurements, and
optionally wherein the at least one static ECG electrode is integrated in a body-wearable unit.

7. A system (10) as claimed in any of claims 1-6, wherein the position guidance function is based at least in part on acquiring ultrasound data representative of an area containing at least a portion of the ribs of the patient, and wherein the atlas dataset comprises reference ultrasound data pertaining to the ribs for different locations.

8. A system(10) as claimed in any of claims 1-7, wherein the position guidance function is based at least in part on collecting ultrasound data of the heart and preferably is based on use of an image registration procedure to determine a position offset of the probe with respect to one or more of the target ECG electrode locations.

9. A system (10) as claimed in claim 8, wherein the acquired ultrasound data includes ultrasound image data, and wherein the system stores a reference ultrasound image view associated with each target ECG electrode location, and wherein the image registration procedure comprises performing registration between acquired ultrasound image data and a reference ultrasound image view for a given target location.

10. A system (10) as claimed in any of claims 1-9, wherein the position guidance function has two modes:
a first mode in which the ultrasound sensing means is controlled to acquire ultrasound data at a first depth level in the body, for acquiring data representative of at least a region of the ribs of the subject, and wherein the position guidance is based on atlas data pertaining to rib locations; and
a second mode in which the ultrasound sensing means is controlled to acquire ultrasound data at a second, deeper, depth level in the body, for acquiring ultrasound data representative of the heart, and preferably wherein the position guidance is based on an image registration procedure, based on atlas data pertaining to the heart.

11. A system (10) as claimed in claim 9 and claim 10, wherein the controller is configured to recurrently perform a mode selection procedure, wherein
the position guidance function operates in the second mode if at least a portion of the reference ultrasound image view for the given target location is within the field of view of the acquired ultrasound data; and
the guidance function operates in the first mode if no part of the reference ultrasound image view for the target location in question is found within the field of view of the acquired ultrasound data.

12. A system (10) as claimed in any of claims 1-11, wherein the system is configured to guide the user to position the probe in a first set of target electrode positions for performing a first set of ECG measurements, and to adjust the stored target electrode locations for a second set of ECG measurements based on ECG measurement data acquired in the first set of ECG measurements.

13. A system (10) as claimed in any of claims 1-12, wherein the controller is configured in at least one operation mode to acquire ultrasound data continuously or at regular intervals, and wherein the position guidance function comprises a continuous or recurrent feedback loop between acquired ultrasound data and generated position guidance information.

14. A method for guiding a user in positioning a medical sensing probe (12) relative to a subject's body, the probe comprising an ultrasound sensing means (18) and at least one ECG electrode (22), and the method comprising:
acquiring ultrasound data using the ultrasound sensing means;
accessing an atlas dataset (28) storing reference ultrasound data associated with each of a plurality of possible locations of the probe relative to the body;
accessing a datastore (30) storing a plurality of reference electrode locations relative to the body for different ECG measurements, and
implementing a position guidance function configured to generate guidance information for a user for positioning the probe to one or more of the reference electrode locations based on the acquired ultrasound data, and based on reference to the atlas dataset.

15. A method as claimed in claim 14, wherein the guidance function is configured to generated guidance information for guiding the user to position the probe (12) through a predefined set of reference electrode locations, for acquiring a predefined set of ECG measurements.
